# EUROPEAN PATENT APPLICATION

(11) **EP 2 402 457 A1**
(43) Date of publication of application: **04.01.2012**
(21) Application number: 10745804.4
(22) Date of filing: 29.01.2010
(51) Int. Cl.: C12Q 1/68

(54) **DETECTING PROBES AND LIQUID PHASE CHIPS FOR BRAF GENE MUTATION AND DETECTING METHODS THEREOF**

(30) Priority: 24.02.2009 CN 200910037357
(71) Applicant: Guangzhou Surexam Bio-Tech Co., Ltd., Guangdong 510663 (CN)
(72) Inventor: XU, Jiasen, Guangzhou Guangdong 510663 (CN); ZHU, Zeyao, Guangzhou Guangdong 510663 (CN); CHEN, Ling, Guangzhou Guangdong 510663 (CN)
(74) Representative: von Kreisler Selting Werner
(86) International application number: PCT/CN2010/070425
(87) International publication number: WO 2010/097020

(57) **Abstract**

Probes, liquid chips and method for detecting BRAF exon 15 mutations are disclosed. The liquid chip for detecting BRAF exon 15 mutations comprises: microspheres coupled with amino-substituted wild-type probes specific for exon 15, and microspheres coupled with amino-substituted mutant-type probes specific for exon 15; primers for amplifying target sequences enriched for mutant alleles of BRAF exons 15 and biotin-labeled at the terminal. The method of this invention is rapid and accurate in detection, and easy in operation, whereby the detection efficiency is greatly improved.

Sequence Listing

<110>  GUANGZHOU SUREXAM BIO-TECH CO., LTD.
<120>  Probes, liquid chips and methods for detecting BRAF gene mutations

<160> 7
<170> PatentIn version 3.1

<210> 1
<211> 23
<212> DNA
<213> artificial sequence
<400> 1
tctagctaca gtgaaatctc gat                    23

<210> 2
<211> 23
<212> DNA
<213> artificial sequence
<400> 2
ctagctacag tgaaatctcg atg                    23

<210> 3
<211> 23
<212> DNA
<213> artificial sequence
<400> 3
tctagctaca gagaaatctc gat                    23

<210> 4
<211> 23
<212> DNA
<213> artificial sequence
<400> 4
ctagctacag agaaatctcg atg                    23

<210> 5
<211> 23
<212> DNA
<213> artificial sequence
<400> 5
tcataatgct tgctctgata gga                    23

<210> 6
<211> 23
<212> DNA
<213> artificial sequence
<400> 6
tagcctcaat tcttaccatc cac                     23

<210> 7
<211> 23
<212> DNA
<213> artificial sequence
<400> 7
acaactgttc aaactgatgg gac                     23

## Description

### FIELD OF THE INVENTION

The present invention relates to the field of molecular biology, and in particular to probes, liquid chips and methods for detecting BRAF gene mutations.

### BACKGROUND OF THE INVENTION

The full name of the BRAF gene is v-raf murine sarcoma viral oncogene homolog B1. The BRAF gene is located at chromosome 7q34, and its functional coding region consists of 2510 base pairs. The BRAF gene codes for the serine/threonine kinase in the MAPK pathway, and the serine/threonine kinase transduces signals from RAS to MEK1/2, thereby regulates various cellular processes, such as cell growth, cell proliferation and cell apoptosis. It is generally agreed today that the T1799A mutation in BRAF exon 15 leads to a valine-to-glutamate substitution at residue 600 (V600E) of the BRAF protein, and further activates BRAF kinase and leads to MAPK pathway activation and unlimited cell proliferation and division. BRAF gene mutations in other sites do not occur frequently. It is reported that the BRAF mutation rate is about 15%, and over 90% of mutations are V600E mutation in exon 15.

At present, BRAF gene analysis in many laboratories is low in detection sensitivity and specificity. The existing methods for detecting BRAF gene mutations mainly include a PCR sequencing method and a Real-time PCR method. The PCR sequencing method has an advantage that the mutation scope and mutation type can be determined, and it is the most widely used detection method, but the sensitivity of the sequencing method is only 20% - 25%. This is far from meeting the demand of practical use, and in particular, for somatic mutations of heterogeneity in tumor cells, the low sensitivity leads to missing detection of mutations. Meanwhile, the sequencing method is poor in timeliness and complicated in operation. Thus, for clinic detection with high timeliness and high sensitivity requirement, the shortcoming of the sequencing method is already highlighted. The sensitivity of the Real-time PCR method can reach to 1% -2%, thus it has high detection efficiency and high timeliness. However, there is a high rate of false positive in practice. Luminex Corporation in USA develops the technique of suspension liquid chip with microspheres as carrier. This technique utilizes polystyrene microspheres as reaction carrier, and a fluorescence detector as a detection platform to detect the multiple analytes of high throughput of biological macromolecules such as nucleic acid and protein. During the preparation process of microspheres, different proportions of red and infrared fluorophores are interally dyed to obtain 100 kinds of microspheres with different color code. Each bead set can be coated with different protein or nucleic acid molecules specific to a particular analyte. Report molecule is biotin-labeled and dyed with high sensitive fluorophores. These microspheres, samples, report molecules, and fluorescent markers form a complete microsphere detection system to be read by the Luminex system.

The Luminex system stimulates the red laser and the green laser respectively for the detection of microsphere system. The red laser detects the intensity of red fluorescence on the microsphere surface, classified and numbered according to different colors of the microspheres so as to determine the type of reaction. The fluorescence intensity of the fluorescent markers in the samples are detected by the green laser, the types of the microspheres and the quantities of the fluorescent markers detected are automatically analyzed by Luminex and computer, thereby the concentration of each target of the samples is determined. Therefore, the liquid chip is qualified for the requirement of high throughput detection, and has the advantages of quick and accurate detection, high sensitivity, good specificity and reproducible results.

### SUMMARY OF THE INVENTION

It is a first object of the present invention to provide probes for detecting BRAF exon 15 mutations.

The probes for detecting BRAF exon 15 mutations comprise:
a wild-type probe of any one selected from SEQ ID NO.1 - SEQ ID NO.2 specific for exon 15; and a mutant-type probe of any one selected from SEQ ID NO.3 - SEQ ID NO.4 specific for exon 15.

It is a second object of the present invention to provide a liquid chip for detecting BRAF exon 15 mutations.

To achieve the above object, the technical solution is as follows:
A liquid chip for detecting BRAF gene mutations comprises:
   (A) microspheres coupled with amino-substituted wild-type probes of any one selected from SEQ ID NO.1 - SEQ ID NO.2 specific for exon 15, and microspheres coupled with amino-substituted mutant-type probes of any one selected from SEQ ID NO.3 - SEQ ID NO.4 specific for exon 15; wherein a spacer is connected between the nucleotide sequence of each kind of the above-mentioned probes and the amino group, and microspheres coupled with different probes have different color codes; and
   (B) primers for amplifying target sequences enriched for mutant alleles of BRAF exons 15, wherein the primers have restriction site, and the target sequence is biotin-labeled at the terminal.

The spacer above-mentioned is the sequence used for separating the specific probe from the microsphere surface or placing the specific probe into the hydrophilic environment. By providing an appropriate length of spacer sequence between the probe and the amino group, the steric hindrance is reduced, and the efficiency of hybridization and the specificity of hybridization are improved. The familiar spacer sequences include poly (dT), oligomer polyethylene glycol and (CH2)n spacer (n≥3), such as (CH2)12, (CH2)18, etc. In addition, if the disturbing of poly (Da) exists, it can also use the poly (TTG) to be the spacer. Preferably, the spacer is an oligonucleotide consisting of 5 to 30 deoxythymidylates.

Preferably, the primers for amplifying target sequences enriched for mutant alleles of BRAF exons 15 comprise the first PCR primers with restriction site and the second PCR primers, and at least one of the primers is biotin-labeled at the terminal. More preferably, the first PCR primers are SEQ ID NO.5 and SEQ ID NO.6, the second PCR primers are SEQ ID NO.5 and SEQ ID NO.7, and at least one of the primers is biotin-labeled at the terminal.

It is a further object of the present invention to provide a method for detecting BRAF exon 15 mutations, which is rapid and accurate in detection, and easy in operation.

A method for detecting BRAF exon 15 mutations, using the above-mentioned liquid chip for detecting BRAF exon 15 mutations, comprises the following steps:
(1) performing a first PCR amplification for DNA samples by using PCR primers with restriction site;
(2) performing restriction digestion to products obtained from the first PCR amplification of the step (1);
(3) performing a second PCR amplification for the mutant-type BRAF gene by using products obtained after the restriction digestion as template such that second PCR amplification products with mutant alleles of exon 15 are obtained, and the second PCR amplification products being biotin-labeled at the terminal;
(4) hybridizing the second PCR amplification products to the corresponding probes coupled to the microspheres;
(5) performing reaction by adding streptavidin-phycoerythrin after the hybridization reaction of the step (4), and then performing signal detection.

The advantages of the invention are as follows:
(1) By using the liquid chip for detecting BRAF exon 15 mutations, the detection results are more specific, sensitive, and with more than 99% accuracy.
(2) The detection method of the present invention is simplified in operation, which avoids uncertainty factors during the complicated operation process, and hence the accuracy of detection is highly improved. This method can be used for analyzing DNA in tissue samples and DNA in body fluid.
(3) The time required in the detection method of the present invention is much less than that of normal sequencing technique.
(4) The present invention uses the method of introducing restriction site and then enriching by restriction digestion so as to amplify the target mutant-type sequence and then to be used in detection, which prevents the large amount of wild-type sequences in the products from disturbing the detection results.

### BRIEF DESCRIPTION OF THE DRAWINGS

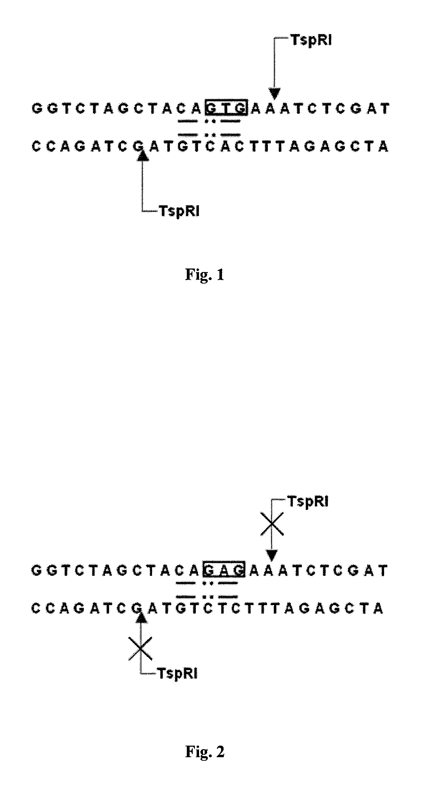

Fig. 1 is a schematic diagram showing the wild-type BRAF gene exon 15 enriched by restriction digestion of TspRI;
Fig. 2 is a schematic diagram showing the mutant-type BRAF gene exon 15 enriched by restriction digestion of TspRI.

### DETAILED DESCRIPTION OF THE PREFERRED EMBODIMENT

BRAF gene mutation is a kind of somatic mutation, the detection of which is disturbed by a large number of wild-type genes. Therefore, in addition to design probes for detecting BRAF gene mutations, it is a further key problem on how to detect a very small number of BRAF gene mutations that are disturbed by a large number of wild-type genes. Two pairs of PCR primers are designed, wherein the first PCR primers have restriction sites. In order to enable the final PCR products to be biotin-labeled, the primers may be biotin-labeled at the terminal or the biotinylated dCTP can be used in incorporating during the second PCR reaction. In the embodiments of the present invention, primers with the terminal biotin-labeled are preferred. The present invention uses the method of mutant-enriched, wherein a first PCR amplification is used to amplify the wild-type sequences and the mutant-type sequences simultaneously, then a large number of wild-type genes are removed through restriction digestion, and a second PCR amplification is used to amplify the mutant-type genes so as to enrich the mutant-type sequences, and finally the BRAF gene exon 15 mutations are detected by using the liquid chip technique.

The main materials required for the assay
Solution Formulation : Coupling buffer ( pH4.5 ) : 0.1mol/LMES ( Sigma M-2933 )
Washing buffer : 0.2ml/L Tween-20 ( Sigma P-9416 ) , 1g/L SDS ( Sigma L-4390 )
TE buffer ( pH8.0 )( Storage solution ) : 10mmol/L Tris ( Sigma 337501 ), 1mmol/L EDTA ( Sigma E-5134 )
2 Tm hybridization buffer

| Reagent | Source | Final concentration | Serving size per 250ml |
|---|---|---|---|
| 1MTris-HCl, pH8.0 | SigmaT3038 | 0.2M | 50ml |
| 5M NaCl | Sigma S5150 | 0.4M | 20ml |
| Triton X-100 | Sigma T8787 | 0.16% | 0.4ml |

Filtered and stored at 4 °C.

### EMBODIMENT 1

### Preparation of liquid chip for detecting BRAF exon 15 mutations

### 1. The probes and microsphere coupling

Specific oligonucleotide probes were designed for detection of the wild-type and mutant-type of BRAF exon 15. A spacer with 15 to 30 deoxythymidylates (normally 10 deoxythymidylates, and the effect is identical to that of 5 to 30 deoxythymidylates) is added during the synthesis of oligonucleotide probes. The coupling of each kind of the microspheres comprises the following steps:
(1) suspend the stock uncoupled microspheres (purchased from Luminex Corporation) by vortex;
(2) transfer 8µl of the stock microspheres, which contains a total of 0.8 10⁵ to 1.2 10⁵ microspheres, to a 0.5ml microcentrifuge tube;
(3) pellet the microspheres by microcentrifugation at 10,000 rpm for 3 min, and remove the supernatant;
(4) add 10µl of coupling solution (pH 4.5), and mix evenly by vortex;
(5) add 2µl of 2pmol/ul probe working solution;
(6) add 2.5µl of 5mg/ml EDC (1-(3-Dimethylaminopropyl)-3-ethylcarbodiimide hydrochloride) working solution, and incubate at 25 °C for 30min; and repeat this step once;
(7) add 0.2ml of washing buffer, mix evenly by vortex and then pellet by microcentrifugation at 12,000g for 3 min, and remove the supernatant; repeat this step once;
(8) add 500µl of TE buffer, and mix evenly by vortex;
(9) pellet by microcentrifugation at 12,000g for 3 min, and remove the supernatant;
(10) add 20µl of TE buffer, and stored at 2-8 °C.

The prepared liquid chips for detecting BRAF exon 15 mutations include: two kinds of microspheres coupled with wild-type probes and two kinds of microspheres coupled with mutant-type probes, wherein a liquid chip with a pair of microspheres coupled with wild-type probes and mutant-type probes is used for the detection.
A. for the microspheres coupled with probes, the nucleotide sequence for each probe is as follows:

| Microspheres No. | Probes Names | Probe nucleotide sequences | SEQ.ID NO. |
|---|---|---|---|
| 38 | B15W1 (Wild-type) | 5'NH2-TTTTTTTTTT TCTAGCTACAGTGAAATCTCGAT 3' | 1 |
| 63 | B15W2 (Wild-type) | 5’NH2-TTTTTTTTTT CTAGCTACAGTGAAATCTCGATG 3’ | 2 |
| 14 | B15M1 (Mutant-type) | 5'NH2-TTTTTTTTTT TCTAGCTACAGAGAAATCTCGAT 3' | 3 |
| 25 | B15M2 (Mutant-type) | 5’NH2-TTTTTTTTTT CTAGCTACAGAGAAATCTCGATG 3’ | 4 |

B: Primers: the primers for amplifying the target sequences enriched for mutant alleles of the BRAF exon 15 to be tested, and the amplified target sequence can be biotin-labeled at the terminal, the nucleotide sequence of each primer is as follows:

| Primers Name | nucleotide sequences | First or Second round PCR | SEQ.ID NO. |
|---|---|---|---|
| B15F | 5' TCATAATGCTTGCTCTGATAGGA 3' | First round and Second round | 5 |
| B15R1 | 5' TAGCCTCAATTCTTACCATCCAC 3' | First round | 6 |
| B15R2-bio | 5'biotin-  ACAACTGTTCAAACTGATGGGAC 3' | Second round | 7 |

### EMBODIMENT2

Clinic samples are detected by using the liquid chip for detecting BRAF gene mutations
1. Preparation of the samples to be detected
Nucleic acid is extracted according to AxyPrep extraction kit instructions to obtain 10 cases of DNA samples to be detected.
2. PCR amplification and Mutant-enrichedof the samples to be detected
**1). The first PCR amplification**

**PCR reaction system**

| Reaction system | Per reaction (µl) |
|---|---|
| Sterile ddH₂O | 28.8 |
| 5 Buffer | 10 |
| 2.5mM dNTP Mixture | 2 |
| MgCl₂ 25mM | 5 |
| Primer F : B15F(10uM) | 1 |
| Primer R : B15R1(10uM) | 1 |
| Taq polymerase ( 5U/ul ) | 0.2 |
| DNA Templates | 2 |
| Total volume | 50 |

PCR Reaction condition :

| **Steps** | **Temperature** | **Time** | **cycle numbers** |
|---|---|---|---|
| Initial denaturation | 94°C | 3 min | 1 |
| Denaturation | 94°C | 20 sec | 25 |
| Anneal | 58°C | 30 sec | |
| Extension | 72 °C | 20 sec | |
| Final extension | 72 °C | 10 min | 1 |
| Preservation | 4°C | ∞ | 1 |

**2). TspRI restriction digestion of PCR Products:**
Reaction system is as follow:

| Sample system (Incubated at 60°C for 2hr) | Per reaction ( µl ) |
|---|---|
| 10 Buffer C | 5 |
| TspRI endonuclease ( 10U/ul ) | 0.5 |
| BSA | 0.5 |
| PCR products | 10 |
| Sterile ddH₂O | 34 |
| Total volume | 50 |

**3). The second PCR amplification**
PCR reaction system:

| Reaction system | Per reaction (ul) |
|---|---|
| Sterile ddH₂O | 28.8 |
| 5 Buffer | 10 |
| 2.5mM dNTP Mixture | 2 |
| MgCl₂ 25mM | 5 |
| Primer F : B15F(10uM) | 1 |
| Primer R : B15R2-bio(10uM) | 1 |
| Taq polymerase ( 5U/ul ) | 0.2 |
| DNA Templates | 2 |
| Total volume | 50 |

PCR Reaction condition :

| Steps | Temperature | Time | cycle numbers |
|---|---|---|---|
| Initial denaturation | 94 °C | 3 min | 1 |
| Denaturation | 94 °C | 20 sec | **30** |
| Anneal | 60°C | 30 sec | |
| Extension | 72 °C | 20 sec | |
| Final extension | 72 °C | 10 min | 1 |
| Preservation | 4°C | ∞ | 1 |

**3. Hybridization and Luminex detection**
(1) preparation of microspheres working solution: take the microspheres (in Embodiment 1) coupled with amino-substituted wild-type probes of SEQ ID NO.1 and SEQ ID NO.2 specific for exon 15, and the microspheres (in Embodiment 1) coupled with amino-substituted mutant-type probes of SEQ ID NO.3 and SEQ ID NO.4 specific for exon 15, wherein the amino-substituted wild-type probes and the amino-substituted mutant-type probes form different probe set arrays; these microspheres are evenly mixed by vortex, then the desired microspheres are added into the sterilized microfuge tube (0.5ul per reaction, and 2000 microspheres per ul for each kind of microsphere), then add 1.5X hybridization buffer (32.5ul per reaction) and TE buffer (14ul per reaction), and this is the microspheres working solution;
(2) according to the sample condition, the marker pen is used to mark the 96 well plate (hybridization plate) (negative control wells marked as "N"), and then add 47ul of microspheres working solution into each well;
(3) add 3ul of corresponding hybridization sample (the second PCR products) to the sample wells; add 3ul ofTE buffer to the "N" well;
(4) put the hybridization plate into the PCR instrument, proceed with denaturation for 3min at 95 °C; and then hybridization at 55 °C - 60 °C for 15min;
(5) add the SA-PE working solution (10ug/ml) into the hybridization plate after hybridization, 25 ul for each well;
(6) put the hybridization plate into the PCR instrument for reaction for 5min at 60 °C;
(7) set instrument parameters according to Luminex instrument (read 100 microspheres for each sample, the reading time is 25 seconds). The hybridization plate is placed in the tray of Luminex instrument, and the sample MFI values are detected.

**4. Detection results and data analysis**
The reaction products are detected by the Luminex analyzer. Its detection results are indicated in Table 1. The cut-off value is Mean Fluorescence Intensity (MFI) value 100. While the MFI value of mutant-type probes is beyond 100, it is judged that the sample has V600E mutations of exon 15, or else the sample is judged as the wild-type of exon 15. According to the above criteria, there are exon 15 mutaions in two samples (samples No. 3 and No. 5) among ten samples detected in this embodiment.
The test results reveal that the analysis result of the detection of liquid chip is identical to that of the detection of direct sequencing, that is, there are two V600E point mutations of exon 15 (Samples No. 3 and No. 5). This shows that the method for detecting BRAF gene mutations by using free nucleic acid is feasible, and also shows that the liquid chip and detection method provided by the invention is stable and reliable.
As shown in Table 1 and Table 2, the detection results and analysis results of different probe set arrays are identical, that is, there are two V600E point mutations of exon 15 (Samples No. 3 and No. 5). This shows that the two probe set arrays provided in this invention (SEQ ID NO. 1 and SEQ ID NO.3; SEQ ID NO. 2 and SEQ ID NO. 4) for the method for detecting BRAF gene mutations are feasible, and also shows that the probes provided are stable and reliable.

**Table 1: Detection results of the sample**

| Probe set array type | NO. | Negative Control | Exon 15 wild-type | Exon 15 mutant-type |
|---|---|---|---|---|
| Wild-type: SEQ ID NO.1; | 1 | 18 | 1486 | 76 |
| | 2 | 14 | 1319 | 79 |
| | 3 | 26 | 470 | 823 |
| Mutant-type: SEQ ID NO. 3 | 4 | 21 | 1342 | 49 |
| | 5 | 19 | 446 | 955 |
| | 6 | 17 | 1387 | 65 |
| | 7 | 24 | 1280 | 71 |
| | 8 | 15 | 1367 | 78 |
| | 9 | 18 | 1471 | 69 |
| | 10 | 25 | 1404 | 53 |
| Wild-type: SEQ ID NO. 2; | 1 | 20 | 1328 | 72 |
| | 2 | 19 | 1395 | 60 |
| | 3 | 14 | 450 | 886 |
| | 4 | 13 | 1321 | 38 |
| | 5 | 18 | 516 | 864 |
| Mutant-type: SEQ ID NO. 4 | 6 | 14 | 1378 | 64 |
| | 7 | 24 | 1402 | 84 |
| | 8 | 15 | 1327 | 47 |
| | 9 | 22 | 1396 | 52 |
| | 10 | 18 | 1424 | 69 |
| Wild-type: SEQ ID NO. 1; | 1 | 25 | 758 | 55 |
| | 2 | 13 | 1025 | 67 |
| | 3 | 26 | 324 | 785 |
| | 4 | 33 | 362 | 54 |
| | 5 | 14 | 424 | 904 |
| Mutant-type: SEQ ID NO. 4 | 6 | 19 | 387 | 56 |
| | 7 | 31 | 457 | 64 |
| | 8 | 25 | 667 | 59 |
| | 9 | 25 | 561 | 68 |
| | 10 | 18 | 511 | 70 |
| Wild-type: SEQ ID NO. 2; | 1 | 30 | 458 | 49 |
| | 2 | 19 | 495 | 88 |
| | 3 | 34 | 335 | 907 |
| Mutant-type: SEQ ID NO.3 | 4 | 23 | 451 | 58 |
| | 5 | 17 | 321 | 851 |
| | 6 | 25 | 1058 | 55 |
| | 7 | 21 | 902 | 71 |
| | 8 | 29 | 657 | 54 |
| | 9 | 32 | 916 | 45 |
| | 10 | 19 | 1241 | 59 |

**Table 2: Analysis results of BRAF gene exon 15 mutations in samples**

| Probe array type | Sample No. | Sequencing results | Detection results of the liquid chip the liquid chip |
|---|---|---|---|
| | 1 | Wild-type | Wild-type |
| | 2 | Wild-type | Wild-type |
| Wild-type: | 3 | V600E point mutation | V600E point mutation |
| SEQ ID NO.1; | 4 | Wild-type | Wild-type |
| | 5 | V600E point mutation | V600E point mutation |
| Mutant-type: | 6 | Wild-type | Wild-type |
| SEQ ID NO. 3 | 7 | Wild-type | Wild-type |
| | 8 | Wild-type | Wild-type |
| | 9 | Wild-type | Wild-type |
| | 10 | Wild-type | Wild-type |
| Wild-type: | 1 | Wild-type | Wild-type |
| SEQ ID NO. 2; | 2 | Wild-type | Wild-type |
| | | V600E point | V600E point |
| Mutant-type: | 3 | mutation | mutation |
| SEQ ID NO. 4 | 4 | Wild-type | Wild-type |
| | 5 | V600E point mutation | V600E point mutation |
| | 6 | Wild-type | Wild-type |
| | 7 | Wild-type | Wild-type |
| | 8 | Wild-type | Wild-type |
| | 9 | Wild-type | Wild-type |
| | 10 | Wild-type | Wild-type |
| | 1 | Wild-type | Wild-type |
| | 2 | Wild-type | Wild-type |
| | 3 | V600E point mutation | V600E point mutation |
| Wild-type: | 4 | Wild-type | Wild-type |
| SEQ ID NO. 1; | 5 | V600E point mutation | V600E point mutation |
| Mutant-type: | 6 | Wild-type | Wild-type |
| SEQ ID NO. 4 | 7 | Wild-type | Wild-type |
| | 8 | Wild-type | Wild-type |
| | 9 | Wild-type | Wild-type |
| | 10 | Wild-type | Wild-type |
| | 1 | Wild-type | Wild-type |
| Wild-type: | 2 | Wild-type | Wild-type |
| SEQ ID NO. 2; | 3 | V600E point mutation | V600E point mutation |
| Mutant-type: | 4 | Wild-type | Wild-type |
| SEQ ID NO. 3 | 5 | V600E point mutation | V600E point mutation |
| | 6 | Wild-type | Wild-type |
| | 7 | Wild-type | Wild-type |
| | 8 | Wild-type | Wild-type |
| | 9 | Wild-type | Wild-type |
| | 10 | Wild-type | Wild-type |

## Claims

1. Probes for detecting BRAF exon 15 mutations, **characterized by** comprising:
a wild-type probe of any one selected from SEQ ID NO.1 - SEQ ID NO.2 specific for exon 15; and a mutant-type probe of any one selected from SEQ ID NO.3 - SEQ ID NO.4 specific for exon 15.

2. A liquid chip for detecting BRAF gene mutations, **characterized by** comprising:
(A) microspheres coupled with amino-substituted wild-type probes of any one selected from SEQ ID NO.1 - SEQ ID NO.2 specific for exon 15, and microspheres coupled with amino-substituted mutant-type probes of any one selected from SEQ ID NO.3 - SEQ ID NO.4 specific for exon 15; wherein a spacer is connected between the nucleotide sequence of each kind of the above-mentioned probes and the amino group, and microspheres coupled with different probes have different color codes; and
(B) primers for amplifying target sequences enriched for mutant alleles of BRAF exons 15, the target sequence being biotin-labeled at the terminal.

3. The liquid chip for detecting BRAF gene mutations of claim 2, **characterized in that** the primers comprise the first PCR primers with restriction site and the second PCR primers, and at least one of the primers is biotin-labeled at the terminal.

4. The liquid chip for detecting BRAF gene mutations of claim 3, **characterized in that** the first PCR primers are SEQ ID NO.5 and SEQ ID NO.6, the second PCR primers are SEQ ID NO.5 and SEQ ID NO.7, and at least one of the primers is biotin-labeled at the terminal.

5. The liquid chip for detecting BRAF gene mutations of any one of claims 2 to 4, **characterized in that** the spacer is an oligonucleotide consisting of 5 to 30 deoxythymidylates.

6. A method for detecting BRAF exon 15 mutations, using the liquid chip for detecting BRAF gene mutations of any one of claims 2 to 5, **characterized by** comprising the following steps:
(1) performing a first PCR amplification for DNA samples by using PCR primers with restriction site;
(2) performing restriction digestion to products obtained from the first PCR amplification of the step (1);
(3) performing a second PCR amplification for the mutant-type BRAF gene by using products obtained after the restriction digestion as template such that the second PCR amplification products with mutant alleles of exon 15 are obtained and the second PCR amplification products being biotin-labeled at the terminal;
(4) hybridizing the second PCR amplification products to the corresponding probes coupled to the microspheres;
(5) performing reaction by adding streptavidin-phycoerythrin after the hybridization reaction of the step (4), and then performing signal detection.

7. The method for detecting BRAF exon 15 mutations of claim 6, **characterized in that** the first PCR primers are SEQ ID NO.5 and SEQ ID NO.6, the second PCR primers are SEQ ID NO.5 and SEQ ID NO.7, and at least one of the primers is biotin-labeled at the terminal.

8. The method for detecting BRAF exon 15 mutations of claim 6, **characterized in that** the hybridization temperature in the step (4) is 55-60°C.
